(19) **Europäisches Patentamt** / **European Patent Office** / **Office européen des brevets**

(11) **EP 4 470 459 A1**

(12) **EUROPEAN PATENT APPLICATION**

(43) Date of publication:
**04.12.2024 Bulletin 2024/49**

(21) Application number: **24177641.8**

(22) Date of filing: **23.05.2024**

(51) International Patent Classification (IPC):
**A61B 5/06** (2006.01)          **A61B 34/20** (2016.01)

(52) Cooperative Patent Classification (CPC):
**A61B 5/066; A61B 5/6851; A61B 5/6852;**
A61B 2034/2061; A61B 2562/0266

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.05.2023 US 202318322334**

(71) Applicants:
- **FBGS Technologies GmbH**
  **07745 Jena (DE)**
- **FBGS International NV**
  **2440 Geel (BE)**

(72) Inventors:
- **VAN ROOSBROECK, Jan**
  **2220 Hallaar (BE)**
- **VLEKKEN, Johan**
  **3590 Diepenbeek (BE)**
- **VAN HOE, Bram**
  **9000 Gent (BE)**
- **VOIGTLÄNDER, Christian**
  **07745 Jena (DE)**
- **LINDNER, Eric**
  **07743 Jena (DE)**

(74) Representative: **Winger**
**Mouterij 16 bus 101**
**3190 Boortmeerbeek (BE)**

(54) **POSITION DETERMINATION OF AN ELONGATED MEDICAL DEVICE TAKING INTO ACCOUNT CONFINEMENT**

(57)      A method and system for determining the position of one or more points of an elongated medical device in a channel inside an object are described. The method comprises obtaining three-dimensional image data of an inside of the object comprising at least data of a channel of interest inside the object, deriving based on the obtained three-dimensional image data derived shape information for at least part of the channel of interest, and deriving, for positions along the channel of interest, a confinement parameter expressing whether the elongated medical device will be confined at those positions in the channel of interest. The method also comprises obtaining measured shape information of an elongated medical device based on optical signals obtained from a multicore fiber coupled to the elongated medical device, for determining shape information of the elongated medical device, and determining a position of one or more points of the elongated medical device with respect to the three-dimensional image data or a portion thereof by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

FIG. 8

EP 4 470 459 A1

**Description**

**FIELD OF THE INVENTION**

The present invention relates to the medical field. More particularly, it relates to methods and systems for determining a position of an elongated medical device (e.g., a catheter or endoscope) inserted in a channel of an object.

**BACKGROUND OF THE INVENTION**

Where possible during surgery or medical examination, one attempts to use minimal invasive surgery or examination rather than open surgery. The latter is advantageous, since it reduces surgical risks as well as pain and it speeds up recuperation after the medical intervention. When applying minimal invasive surgery or examination, often use is made of an elongated medical device such as a guidewire, catheter, endoscope, or other type of elongated medical device that can be inserted in the body.

In order to provide information to the surgeon regarding the procedure, the insertion of the elongated medical device in the body often is done whilst applying medical imaging, in order to assist the surgeon in the procedure, e.g., in deciding where the elongated medical device is currently positioned and what direction it needs to go. Different medical imaging techniques are available, but at least some of them suffer from the fact that hazardous radiation of/in the body is required.

For determining a direction to go or a position where the elongated medical device is positioned, use could also be made of an optical multicore fiber, since the shape of an optical multicore fiber could be determined based on optical signals stemming therefrom. Nevertheless, accuracy of the determination of the shape and position at present is limited, since side effects such as for example twist, limit accurate shape determination and since suitable methods and systems for obtaining position information still need to be improved.

**SUMMARY OF THE INVENTION**

It is an object of the present invention to provide good methods and systems for assisting in guiding an elongated medical device in an object. In some particular embodiments, such methods and systems may be methods and systems for assisting in guiding an elongated medical device in a body of a creature, e.g. a living creature, e.g. during performance of minimal invasive surgery. The elongated medical device may for example be a tubular shaped elongated medical device, a guidewire, a catheter, an endoscope, a gastroscope or other type of elongated medical device that can be inserted in a channel of the body.

The object and optionally one or more advantages can be obtained using methods and systems according to embodiments of the present invention.

In one aspect, a method for determining the position of one or more points of an elongated medical device in a channel inside an object is disclosed. The method comprises

-    obtaining three-dimensional image data of an inside of the object comprising at least data of a channel of interest inside the object,
-    deriving based on the obtained three-dimensional image data derived shape information for at least part of the channel of interest,
-    deriving, for positions along the channel of interest, a confinement parameter expressing whether the elongated medical device will be confined at those positions in the channel of interest,
-    obtaining measured shape information of an elongated medical device based on optical signals obtained from a multicore fiber coupled to the elongated medical device, for determining shape information of the elongated medical device, and
-    determining a position of one or more points of the elongated medical device with respect to the three-dimensional image data or a portion thereof by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

Where in embodiments of the present invention reference is made to shape information, this may in some embodiments refer to curvature information, such as for example bending direction, curvature direction, curvature angle, or another parameter describing a curvature profile or a curvature profile. It may in some examples be curvature information such as a bending direction or curvature direction along the arc length of the path or along the curved path. In some embodiments, shape information may comprise or may be a 3D shape, such as for example 3D coordinates.

In some embodiments, the method may comprise segmenting the obtained three dimensional image data so as to obtain segmented three-dimensional image data of at least the channel of interest. Segmenting may be performed using known techniques in the field.

The multicore fiber may in some embodiments comprise a plurality of cores, whereby a plurality of optical sensing elements are inscribed in the multicore fiber, the plurality of optical sensing elements being positioned along a portion of the length of the multicore fiber.

The object may be a body, e.g. a body of a creature such as a living creature. The channel of interest may be an artery or a blood vessel, but embodiments are not limited thereto and may for example also be another channel in the body of a living creature.

The optical sensing elements may in some embodiments be based on Bragg gratings in combination with wavelength division multiplexing, code division multiplexing or Optical Frequency Domain Reflectometry (OFDR) techniques. Nevertheless, optical measurements based on other techniques or also using other techniques, such as for example making use of Optical Frequency Domain Reflectometry (OFDR) based on Rayleigh backscattering without FBG's, or other FBG or non-FBG measurement technologies also are envisaged in the present invention and embodiments describing the use of Bragg gratings may be equally implemented using such other optical sensing elements and corresponding optical sensing techniques.

In some embodiments, the method furthermore may comprise, based on said determining a position, showing said determined position of one or more points of the elongated medical device with respect to the three-dimensional image data.

Deriving a confinement parameter may comprise indicating that the channel of interest is confined for positions where the channel width is smaller than four times the width of the elongated medical device and selecting these portions for use.

A channel of interest may be considered confined for positions where the channel width is smaller than between 1,5 and 4 times the width of the elongated medical device. The confinement parameter may for example in some embodiments be a ratio in width between the elongated medical device and the channel of interest. In some embodiments, the confinement parameter may be a ratio of the diameter, e.g. average diameter, between the elongated medical device and the channel of interest. The confinement parameter also may be used as a weighting parameter, e.g. in a fitting procedure. Deriving a confinement parameter based on the channel width may be performed using the three-dimensional image data.

At positions along the channel of interest where the channel of interest is not confined according to the confinement parameter, the orientation of the elongated medical device may be derived from the measured shape information measured using the multicore fiber.

Determining a position of one or more points of the elongated medical device may comprise determining a position of one or more points of the elongated medical device based on said mapping for the portion of the channel of interest wherein the elongated medical device is confined and determining a position of one or more additional points of the elongated medical device for points where the elongated medical device is not confined in the channel of interest from the measured shape information.

The portion of the channel of interest wherein the elongated medical device is confined may be a single contiguous portion, and the portion of the channel of interest wherein the elongated medical device is not confined may correspond to free space.

In some embodiments, confined channels of interest may correspond with e.g. artery, whereas non-confined channels of interest may be free space in the heart.

The method may comprise determining a position of the end point of the elongated medical device being in free space, said determining being based on mapping of a portion of the elongated medical device where it is confined in the channel of interest and on the measured shape information for the portion of the elongated medical device where it is not confined.

The portion of the channel of interest wherein the elongated medical device is confined may correspond with two or more non-contiguous regions.

Obtaining the derived shape information of a portion of a channel of interest and/or obtaining the measured shape information of a portion of an elongated medical device may comprise obtaining information of a curvature parameter or a shape information such as 3D coordinates. An example of a curvature parameter may be a curvature direction, bending direction or curvature angle in a point or as function of a length along that portion of a channel of interest or along that portion of an elongated medical device.

Determining a position of one or more points of the elongated medical device with respect to the three-dimensional image data may comprise

- determining, for a plurality of channels of the three-dimensional image data, the difference between at least a portion of the measured shape information and at least a portion of the derived shape information of the channel,

- identifying the channel of the three-dimensional image data showing the smallest difference between the at least portion of the measured shape information and the at least portion of the derived shape information, and
- matching the position of the elongated medical device with the position of the identified channel.

The plurality of channels may represent different branches of a branched channel of interest. Obtaining three-dimensional image data of an inside of the body may comprise capturing a CT scan of the object.

The length of the multicore fiber along which a plurality of optical sensing elements for obtaining the optical signals are positioned may be 60cm or smaller, e.g. a length of 40cm or smaller, e.g. a length of 20cm or smaller.

The multicore fiber may be a fiber comprising a plurality of cores, at least two cores having a plurality of Bragg gratings inscribed, the plurality of Bragg gratings being spaced apart from each other and being positioned along the length of the multicore fiber.

The method furthermore may comprise a data output, an auditive output or a visual output for outputting information regarding one or more positions of the elongated medical device with respect to a coordinate system of the body or an image thereof.

The present invention also relates to a system for determining a position of an elongated medical device in an object, the system comprising

a data input port for obtaining three-dimensional image data of an inside of the object comprising at least data of a channel of interest, and for obtaining optical signals obtained from a multicore fiber coupled to the elongated medical device in the object,

a processor programmed to obtain three-dimensional image data of at least the channel of interest and for deriving based on the obtained three-dimensional image data a derived shape information for at least part of the channel of interest, and for deriving a confinement parameter expressing whether the channel of interest is confined for the elongated medical device at positions along the channel of interest,

the processor being programmed for obtaining a measured shape information of an elongated medical device based on the obtained optical signals, and

the processor furthermore being programmed for determining a position of one or more points of the elongated medical device with respect to the three-dimensional image data or part thereof by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

The system furthermore may comprise a graphical user interface for outputting a position of one or more points of the elongated medical device with respect to at least part of the three-dimensional image. In one aspect, the present invention also relates to a non-transitory computer program product adapted for, when run on a processor, performing the steps of a method for assisting in guiding an elongated medical device in an object, the method comprising

- obtaining three-dimensional image data of an inside of the object comprising at least data of a channel of interest,
- deriving based on the obtained three-dimensional image data a derived shape information for at least part of the channel of interest,
- obtaining measured shape information of an elongated medical device based on optical signals obtained from a multicore fiber coupled to the elongated medical device in the object, for the elongated medical device when inserted in the object, and
- determining a position of one or more points of the elongated medical device with respect to the three-dimensional image data by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

Obtaining three-dimensional image data of an inside of the object may comprise the actual step of capturing a three-dimensional image of the inside of the object or may be receiving three-dimensional image data from a data source.

It is an advantage of at least some embodiments of the present invention that it is not required to perform three-dimensional imaging of the inside of the object or of the elongated device in the object, e.g. using fluoroscopy, X-ray or CT-scan, during the guiding procedure of guiding the elongated device in the object. The three-dimensional imaging of the inside of the body may be for example previously recorded. The latter results in more easy guiding procedures for guiding devices in objects, for example in more easy guiding procedures for guiding medical devices in bodies, e.g. during minimal invasive surgical procedures.

It is an advantage of embodiments of the present invention that a visualisation is provided of the insertion of the elongated device in the object, thus allowing for example medical staff to be assisted during minimal invasive surgical procedure. The method may assist the medical staff in deciding whether or not an elongated medical device such as a catheter is positioned correctly, thus allowing to perform good or improved medical procedures.

Mapping at least a portion of the measured shape information of the elongated medical device with at least a portion of the derived shape information for at least part of the channel of interest may comprise determining a portion of the channel of interest in which the elongated medical device is confined and mapping the measured shape information, e.g. curvature profile, and the derived shape information, e.g. curvature profile, for that determined portion of the channel of interest.

It is an advantage of embodiments of the present invention that by using only portions of the channel of interest in which the elongated device is confined, the accuracy of the obtained results is high, resulting in good or improved methods.

Determining a portion of the channel of interest in which the elongated device is confined in some embodiments may comprise identifying portions of the channel of interest where the channel width is smaller than four times the width of the elongated device and selecting these portions for use.

In some embodiments, determining a portion of the channel of interest in which the elongated device is confined may comprise identifying a portion wherein the channel of interest has a channel width smaller than three times the width of the elongated device, e.g. identifying a portion wherein the channel of interest has a channel diameter being smaller than three times the diameter of the elongated device.

Such identifying a portion of the measured shape information not used for the mapping based on historical data of the measured shape information may comprise selecting the portion based on a variation of the measured shape information in historical data being larger than a predetermined threshold. Mapping may for example be performed only for measured shape information wherein the historical data for that position of the measured shape information shows a variation of less than a predetermined percentage.

The portion of the channel of interest wherein the elongated device is confined may be two or more, non-contiguous portions of the elongated device. In intermediate portions, the elongated device may be more prone to having different shapes.

In embodiments according to the present invention, obtaining the derived shape information, e.g. curvature profile of a portion of a channel of interest or 3D coordinates, and/or obtaining the measured shape information, e.g. curvature profile of a portion of an elongated device or 3D coordinates, comprises obtaining such shape information, e.g. information of a curvature parameter or 3D coordinates, as function of a length along that portion of a channel of interest and/or along that portion of an elongated device respectively.

Determining a smallest difference between the at least portion of the measured curvature profile and the at least portion of the derived curvature profile may correspond with determining a least square difference between the derived curvature profile and the measured curvature profile.

It is an advantage of embodiments of the present invention that the length of the measurement section for measuring the shape of the multicore fiber and hence the elongated medical device can be short, e.g. substantially shorter than the length of the elongated medical device that is inserted in the body.. It is an advantage of embodiments of the present invention to provide accurate methods and systems for determining an insertion length of an elongated medical device or a position of an elongated medical device or a tip thereof in an object, e.g., a body of a living creature. The elongated medical device may for example be a tubular shaped elongated medical device, a guidewire, a catheter, an endoscope, a gastroscope or other type of elongated device that can be inserted in the body.

It is an advantage of embodiments of the present invention that determination of the insertion length or determination of a position of an elongated medical device can be determined with high accuracy, e.g., with a resolution higher than determined by the distance between the plurality of Bragg gratings in the multicore fiber used in the measurement system.

It is an advantage of embodiments of the present invention that shape and/or position of an elongated medical device using a multicore fiber with gratings, e.g., fiber Bragg gratings (FBG) can be accurately determined.

It is an advantage of embodiments of the present invention that accurate positional and shape information regarding an elongated medical device can be obtained, based on optical measurements in a multicore fiber.

It is an advantage of embodiments of the present invention that measurements of positional and shape information can be fully based on optical measurements in a multicore fiber, since all necessary information can thus be obtained using a single measurement technique, so that no correlation needs to be found between different measurement techniques. Nevertheless, although not strictly required in all embodiments, in some embodiments still use may be made of other techniques, such as for example imaging techniques like a CT scan for identifying a known shape, as will be described further below.

In some embodiments, the multicore fiber for insertion into the elongated medical device may be inserted such that a position of the tip of the multicore fiber corresponds with a position near the tip of the elongated medical device.

The present invention also relates to methods and systems for determining confinement information for one or more points on an elongated device, e.g. a medical elongated device such as for example a catheter. Methods and systems may for example allow determining whether the elongated object at that point of interest is in a confined channel inside the object, in a broad channel inside the object or in an open space in the object. It thereby is an advantage that no additional information from other technologies are required, e.g. no imaging techniques are required, but that such information can be obtained based on optical signals obtained from a multicore fiber coupled to the elongated medical device.

One or more advantages can be obtained using methods and systems according to embodiments of the present invention.

In one aspect, a method for determining confinement information of an elongated device for at least one position of interest in the body, the method comprising

- for a plurality of conditions of the elongated device, obtaining measured shape information of the elongated device for at least one position of interest in the body based on optical signals obtained from a multicore fiber coupled to the elongated medical device,
- determining, based on a comparison of the obtained measured shape information of the elongated device at said at least one point of interest measured for the plurality of conditions, confinement information of said elongated device for that at least one position of interest in the body.

In the method, determining confinement information based on a comparison of obtained shape information may comprise

- quantifying a variation in the obtained measured shape information of the elongated device at the at least one position of interest in the body for the plurality of conditions, and
- deriving based thereon said quantification the confinement information.

Quanityfing a variation in the obtained measured shape information measured for the plurality of conditions may comprise determining a variance or standard deviation in the obtained shape information for the different measurements, and/or may comprise determining a difference between the extremes of the obtained shape information for the different measurements, etc.

Where in embodiments of the present invention, reference is made to a plurality of conditions of the elongated device, reference may be made to measurements performed at different moments in time, optionally after movement of the elongated device, and/or reference may be made to measurements performed during insertion or withdrawal of the elongated device, using e.g. optical signals stemming from different positions along the elongated device corresponding with said at least one position of interest in the body.

Where in embodiments of the present invention reference is made to shape information, this may in some embodiments refer to curvature information, such as for example bending direction, curvature direction, curvature angle, or another parameter describing a curvature profile or a curvature profile. It may in some examples be curvature information such as a bending direction or curvature direction along the arc length of the path or along the curved path. In some embodiments, shape information may comprise or may be a 3D shape, such as for example 3D coordinates.

The multicore fiber may in some embodiments comprise a plurality of cores, whereby a plurality of optical sensing elements are inscribed in the multicore fiber, the plurality of optical sensing elements being positioned along a portion of the length of the multicore fiber.

The body may be the volume of an object or may be e.g. a body of a creature such as a living creature. The channel of interest may be an artery or a blood vessel, but embodiments are not limited thereto and may for example also be another channel in the body of a living creature.

The optical sensing elements may in some embodiments be based on Bragg gratings in combination with wavelength division multiplexing, code division multiplexing or Optical Frequency Domain Reflectometry (OFDR) techniques. Nevertheless, optical measurements based on other techniques or also using other techniques, such as for example making use of Optical Frequency Domain Reflectometry (OFDR) based on Rayleigh backscattering without FBG's, or other FBG or non-FBG measurement technologies also are envisaged in the present invention and embodiments describing the use of Bragg gratings may be equally implemented using such other optical sensing elements and corresponding optical sensing techniques.

In some embodiments, the method furthermore may comprise, based on said determined confinement parameter, indicating a degree of confinement of different points along the elongated device with respect to the body.

Determining confinement information may comprise expressing a degree of confinement of the elongated device at the position of interest in the body. In some embodiments, a channel or area at a point of interest in the body may be referred to as confined when variation on a position based on the measured shape information is smaller than four times the width of the elongated device at that point of interest. In some embodiments, a channel or area at a point of interest in the body may be referred to as confined when a variance or standard deviation or a deviation between extremes is smaller than a predetermined value, e.g. smaller than a factor times the width of the elongated device or smaller than a factor times a value expressing shape information at that position. The factor may be smaller or larger than 1 and may be determined based on the application, e.g. a catheter in artery, a coloscope during coloscopy, etc.

In some embodiments a channel or area may be considered confined in a point of interest if a variation of the measured shape information is smaller than X percent of the measured values, e.g. smaller than 20%, or smaller than 15% or smaller than 10% of the measured values.

A channel of interest may be considered confined for positions where the channel width is smaller than a factor A times the width of the elongated device used, e.g. between 1,5 and 4 times the width of the elongated device. The confinement parameter may for example in some embodiments be a ratio in width between the elongated device and the channel of interest. In some embodiments, the confinement parameter may be a ratio of the diameter, e.g. average diameter, between the elongated device and the channel of interest. The confinement parameter also may be used as a weighting parameter, e.g. in a fitting procedure.

The method may further comprise deriving from the confinement information, whether a certain position in the body may be used as an internal body reference.

The length of the multicore fiber along which a plurality of optical sensing elements for obtaining the optical signals are positioned may be 60cm or smaller, e.g. a length of 40cm or smaller, e.g. a length of 20cm or smaller.

The multicore fiber may be a fiber comprising a plurality of cores, at least two cores having a plurality of Bragg gratings inscribed, the plurality of Bragg gratings being spaced apart from each other and being positioned along the length of the multicore fiber.

The method furthermore may comprise a data output, an auditive output or a visual output for outputting information regarding one or more positions of the elongated medical device with respect to a coordinate system of the body or an image thereof.

For obtaining the shape information of an elongated device at a position of interest in the body, use may be made of the insertion length by which the elongated device is inserted in the body, as well as other techniques. Although not being limited thereto, embodiments of the present invention may make use of techniques as described in European patent applications EP3934504 and/or EP 4233764.

The accuracy of the confinement information may be improved by using additional reference points, e.g. in body references. Confinement information may more accurately be determined when less or no non-confined regions are present between the reference point used and the position where confinement information is to be determined.

The present invention also relates to a system for determining confinement information of an elongated device for at least one position of interest in the body, the system comprising

- a data input port for obtaining, for a plurality of conditions of the elongated device, measured shape information of the elongated device for at least one position of interest in the body based on optical signals obtained from a multicore fiber coupled to the elongated medical device, and
- a processor being programmed for determining, based on a comparison of the obtained measured shape information of the elongated device at said at least one point of interest measured for the plurality of conditions, confinement information of said elongated device for that at least one position of interest in the body.

The processor may be programmed for quantifying a variation in the obtained measured shape information of the elongated device at the at least one position of interest in the body for the plurality of conditions. The processor may be programmed for deriving based on said quantification, the confinement information.

The processor may be programmed for determining a variance or standard deviation in the obtained shape information for the different measurements, and/or may comprise determining a difference between the extremes of the obtained shape information for the different measurements, etc.

The system furthermore may comprise a graphical user interface for outputting confinement information of the elongated device for one or more positions in the body.

In one aspect, the present invention also relates to a non-transitory computer program product adapted for, when run on a processor, performing the steps of the method for determining confinement information as described above.

Additionally or alternatively, the system may comprise a measurement system for obtaining shape information and the measurement system may comprise a multicore fiber with a plurality of Bragg gratings inscribed therein. The plurality of Bragg gratings may be positioned along the length of the multicore fiber. The measurement system thus may comprise a multicore fiber for insertion into the elongated device such that a position of the tip of the multicore fiber corresponds with a position near the tip of the elongated medical device, the multicore fiber comprising a plurality of cores, whereby a plurality of Bragg gratings are inscribed in the multicore fiber, the plurality of Bragg gratings being spaced apart from each other and being positioned along the length of the multicore fiber. The measurement system also may comprise a measurement device for reading out optical signals obtained from a plurality of cores of a multicore fiber as function of the total length of the multicore fiber portion that has been inserted in the body when an elongated device comprising the multicore fiber is inserted into the body. The measurement device may be adapted for deriving, based on the optical signals, shape information of the multicore fiber for deriving therefrom shape information from the elongated medical device wherein the multicore fiber is inserted.

Where in embodiments of the present invention reference is made to shape information, this may in some embodiments refer to curvature information, such as for example bending direction, curvature direction, curvature angle, or another parameter describing a curvature profile or a curvature profile. It may in some examples be curvature information such as a bending direction or curvature direction along the arc length of the path or along the curved path. In some embodiments, shape information may comprise or may be a 3D shape, such as for example 3D coordinates. This may be local information in a certain position along the fiber or elongated device or similar information along port or all of a fiber section or elongated device section.

Where in embodiments of the present invention reference is made to the tip of the multicore fiber, reference may be made to the portion of the multicore fiber closest to the end position inserted in the body with a length smaller than 16cm, e.g., smaller than 12cm, e.g., smaller than 8cm, e.g., smaller than 6cm.

In embodiments of the present invention, the measurement system may comprise an optical source for irradiating the multicore fiber.

In embodiments of the present invention, the measurement system may comprise one or more optical detectors for detecting a signal, after interaction with the plurality of Bragg gratings, from the plurality of cores.

The measurement device may comprise a processor for processing the different optical signals and for deriving therefrom the shape and/or position information.

In embodiments of the present invention, the multicore fiber may comprise a central core and a plurality of further cores, positioned outside the centre. It is to be noted that the central core is not strictly required.

Embodiments of the present invention advantageously may be used in bodies wherein the channels monitored are substantially static. For example, embodiments may advantageously be used for providing positional information in blood vessels, since the position of the blood vessels does not change with respect to the body. The catheter may be inserted in a blood vessel.

Another example may be for providing positional information in intestines in the human or animal body, whereby a tubular medical device is used for surgery or medical examination. Still another example may be for providing positional information in respiratory tracts, e.g., during intubation.

Where in embodiments of the present invention reference is made to an elongated medical device, reference thus for example may be made to a catheter for cardiovascular applications, to an endoscope, to a gastroscope, to a catheter for intubation in respiratory tracts, to a catheter for brain surgery, etc.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

**BRIEF DESCRIPTION OF THE DRAWINGS**

FIG. 1 illustrates a guiding means as can be used in a measurement system according to an embodiment of the present invention.

FIG. 2 illustrates induced curvature due to insertion of a catheter in the body, as can be used in an embodiment according to the present invention.

FIG. 3 illustrates the cross section of a multicore fiber containing 1 center and 6 outer cores.

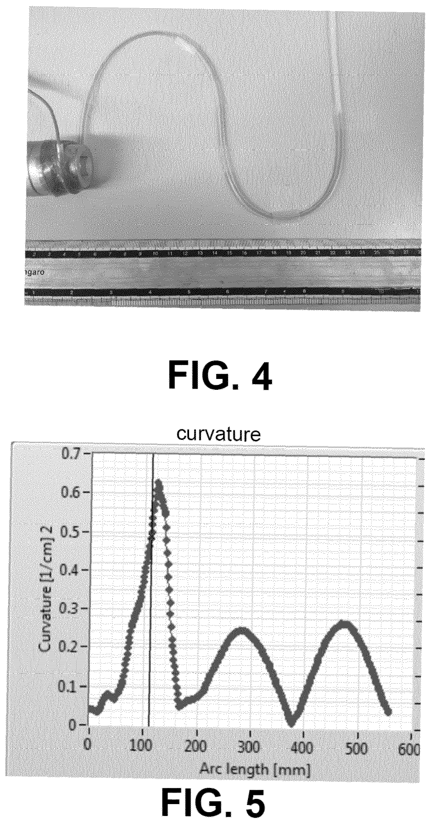

FIG. 4 illustrate an experimental set-up containing a multicore fiber in a catheter sleeve for illustrating features of a measurement system according to an embodiment of the present invention. At the left side on this figure, a guiding means consisting of a Teflon tube bended over a metal cylinder with a radius of 1,6cm is applied. The guiding means goes over into a sine shaped Teflon tube which represents a blood vessel. The catheter sleeve containing the optical fiber is inserted from the left side passing first the guiding means after which it enters the vessel phantom. On the figure, the catheter sleeve and multicore fiber are inserted over one period of the sine wave. FIG. 5 to FIG. 6 illustrate measurements of an experiment for illustrating features of a measurement system according to an embodiment of the present invention.

FIG. 7 illustrates schematically the orientation of the bending plane of a multicore fiber with respect to an outer core, as used in embodiments of the present invention.

FIG. 8 and FIG. 9 illustrate confinement and non-confinement of an elongated medical device in a channel of interest of an object, as used in embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope. In the different drawings, the same reference signs refer to the same or analogous elements.

## DETAILED DESCRIPTION OF ILLUSTRATIVE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein. Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination. In the description provided herein, numerous specific details are set forth. However, it is understood that embodiments of the invention may be practiced without these specific details. In other instances, well-known methods, structures and techniques have not been shown in detail in order not to obscure an understanding of this description.

Where in embodiments it is indicated that the position of the tip of the multicore fiber corresponds with a position of the tip near the tubular medical device, it is to be noted that this will typically not be perfectly matching, since the tubular medical device typically may have a specially shaped tip, such as for example an ablation tip, which does not allow to enter the fiber until the end of the elongated medical device. Nevertheless, the fiber may be sufficiently far introduced in the elongated medical device so that it provides relevant information from the elongated medical device, e.g., even from the tip thereof. Where in embodiments of the present invention reference is made to the fiber comprising a plurality of cores which are used for obtaining information, it will be clear for the person skilled in the art that the number of cores used does not need to be limiting for the total number of cores present in the fiber. In other words, the number of cores present in the fiber may be larger than the number of cores actually used for the measurement. During measuring, the cores used may also be changed, as well as the number of cores that are used. Where in embodiments reference is made to the plurality of cores, reference thus may typically be made to the cores used for measuring, rather than to the total number of cores that is present in the fiber.

One example of how shape information can be obtained is described below. Nevertheless it is to be noted that other techniques known in the are also may be used. Another example is e.g. described in US patent application US2014/0053654.

In the current example described below, use can be made of measurements of gratings of the end portion, also referred to as the tip, of the optical multicore fiber is used. The tip refers to the portion inserted first in the body. An advantage of such embodiments is that this portion typically is less subject to twist and therefore, the change in curvature direction determined in this way, is substantially more representative of the change in bending direction of the elongated medical device. For determining the curvature as such, a single grating is typically sufficient, whereas for determining the change in curvature direction of the vessel, tract or intestines, typically two gratings, e.g., neighbouring gratings, may be used. The change in curvature direction of the vessel, tract or intestines may typically be determined based on the difference in bending planes for two neighbouring gratings, for example the two gratings positioned closest to the end point or for example the last but one and second last but one gratings. By recording the information as function of a given insertion length of the portion of the multicore fiber that is inserted in the body, accurate shape and/or position information along the length of the multicore fiber can be determined. By systematically recording shape information for the tip over time during the insertion of the elongated medical device, i.e. for different insertion lengths, the full shape of the medical device that has been inserted in the body can be mapped, and thus also of the vessel, tract or intestines can be determined. In these type of measurements, use thus is made of shape information that is previously recorded, within the same insertion or retraction action, but at an earlier moment in time during the insertion or retraction which corresponds to another position of the catheter in the body. Previously recorded optical measurements thereby may typically correspond with measurements corresponding to another insertion length of the fiber in the body.

Basically, a recording is made of the shape information from the tip of the fiber as function of the total length of the portion of the elongated medical device that is inserted in the body. Based on this recording, the shape and/or position information along the length of the multicore fiber that has been inserted in the body can be determined.

Formulated differently, as the shape of the vessel, tract or intestines does not change at a position corresponding with a certain length of insertion of the elongated medical device (due to the fact that the vessels, tracts or intestines do not change shape or position during the measurement procedure), the shape information recorded from the tip of the fiber at different moments in time, i.e. for different insertion lengths, translates into shape information along the length of the inserted elongated medical device that is inserted in the body.

An advantage of using previous data recorded during the same insertion or retraction event is that the resolution of the information obtained, e.g., shape information, is not limited by the interdistance between the gratings along the fiber but can be higher depending on how accurate the insertion length of the multicore fiber in the body can be determined and the speed of insertion and the time interval between the measurements that are performed. These can be selected so that the resolution with which the information is captured can be higher than based on the interdistance between the gratings on the fiber. By for example fitting the curvature profile at the pre-defined shape position, one can thus even determine the insertion length at a sub FBG spacing resolution, i.e. the resolution higher than the one determined by the spacing between the fiber gratings. Shape information alternatively may be gathered using more gratings than only those at the tip of the optical multicore fiber, and/or the shape information is combined with length information which is also determined using optical measurements of the multicore fiber. Embodiments of the present invention may make use of techniques as described in European patent applications EP3934504 and/or EP 4233764.

Length information regarding the portion of the optical multicore fiber that is inserted in the body can be obtained based on detection of a particular shape or curvature induced in the optical multicore fiber at a known position with respect to the entry position of the optical multicore fiber in the body or the entry point of the elongated medical device comprising the optical multicore fiber. Two examples of how such a particular shape or curvature can be obtained are described below.

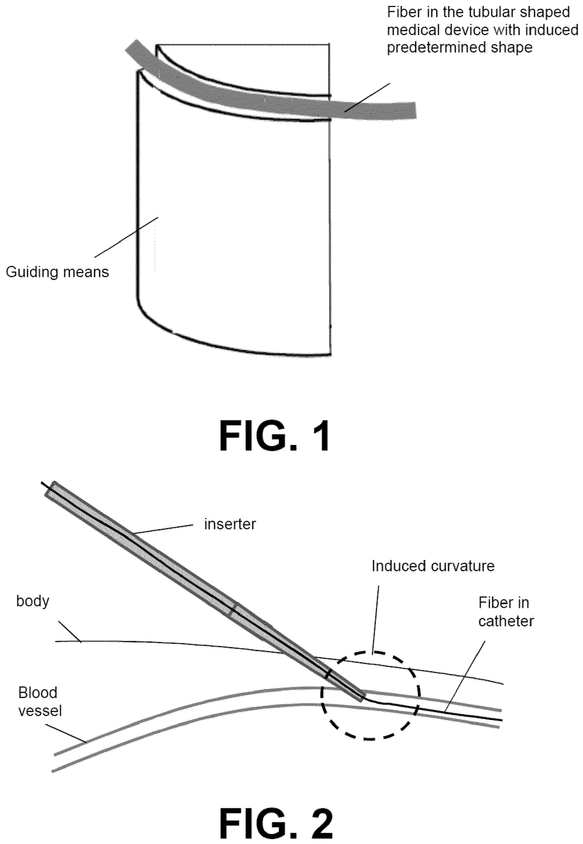

In a first example, the measurement system comprises a guiding means through which the elongated medical device is guided, wherein the guiding means has a known position with respect to the entry point of the elongated medical device into the body. The guiding means furthermore is adapted for inducing in the elongated medical device, a particular shape or curvature such as for example a partial circular shape. By way of illustration, embodiments not being limited thereto, an example of such a guiding means inducing a circular shape is shown in FIG. 1 and Fig. 4.

In Fig. 4, the guiding means imposes a bending to the multicore fiber with a radius of 1,6cm, which corresponds to a curvature of 0,625 (1/cm). Fig. 5 shows the curvature measured along the multicore fiber. As can be seen, a curvature of about 0,625 (1/cm) can be seen at a fiber length position of 120mm, which can be determined as the position where the guiding means applies the biggest curvature to the fiber.

It is an advantage of embodiments of the present invention that the guiding means is not only used as reference for the position but also as a reference for the orientation of the fiber in space, as the orientation of the fiber at the position where the guiding means induces the particular curvature is also known. The latter may allow to create a reference frame wherein both the position as well as the orientation of the fiber is known.

In a second example, the measurement system does not provide a guiding means inducing a particular shape in the elongated medical device, but the particular shape in the elongated medical device is induced by the way the elongated medical device is inserted in the body. The latter is illustrated in FIG. 2, where an inserter is used for inserting the elongated medical device in the body, and wherein a specific curvature is illustrated.

If a guiding means is used for measuring the insertion length or the position, the guiding means e.g., used at the introduction point in the body of creature and also referred to as an introducer, guides the tubular medical device, and as such also the multicore fiber inserted therein, in such a way that it introduces a predetermined shape in the multicore fiber. Recognition of this known shape in the multicore fiber using the optical signals then allows to determine an insertion length or a position of the multicore fiber, hence of the elongated medical device.

As indicated above, alternatively a known shape may be used that is induced in the elongated medical device and hence the multicore fiber by insertion of the elongated medical device in the body and thus by physiological properties of the body. It may for example be a particular shape induced by a shape of a portion of an artery. It is to be noticed that, although such a shape can vary patient by patient, and thus be patient specific, and can vary insertion by insertion, its general shape is sufficiently determined so that it can be recognised and used in the methods and systems according to the present invention. Such a particular shape may be identified using one of a plurality of suitable techniques such as for example imaging techniques like for example fluoroscopy, a CT scan, shape determining methods applied previously, historically obtained shape information or other shape information known from literature. In one example, a CT scan may for example be used for identifying a particular shape of an artery. This particular shape will be induced in the multicore fiber when the catheter and the multicore fiber is introduced in that artery. In another example, no external imaging technique is used, but a particular shape identified during a previous shape determination action, for example but not limited to a shape determination using of methods/systems according to embodiments of the present invention, will be used as known shape. When an insertion action of a tubular medical device, e.g., a catheter, in the body is repeated, this known shape can be used for determining based thereon an insertion length or a position of the tubular medical device.

Deriving an insertion length of the elongated medical device or a position of the elongated medical device based on the known shape information may for example be performed by identifying a location of the known shape information in/on the multicore fiber and deriving therefrom the insertion length or the position.

Embodiments of the present invention may include aligning the multicore fiber and/or elongated medical device at the position of the known shape, with e.g., a representation of the known shape in a coordinate system and deriving based thereon a position of the multicore fiber and/or elongated medical device. The latter may for example be performed by identifying a short shape information fingerprint of the known shape - which act as a fingerprint - in the shape information determined along the length of the multicore fiber. If the known shape is longer than the length of the multicore fiber over which shape information is retrieved, identifying the known shape can be performed by comparing the shape information determined along the length of the multicore fiber with a portion of the longer know shape, e.g., shape information of an artery or path thereof obtained through a CT scan.

Where reference is made to insertion length of the elongated medical device, reference may be made to an insertion length in the body starting from an entrance point in the body but alternatively also reference may be made to a length of insertion with reference to a particular in body reference point such as for example a specific position along the artery. Such a particular in body reference point may also be determined based on an imaging technique such as for example fluoroscopy, a CT scan or information obtained by a previously applied shape information determination technique or based on historical shape information data.

Where reference is made to a position of the elongated medical device or a tip thereof or positional information of the elongated medical device, reference may be made to a position of the elongated medical device with respect to a reference point, such as for example an insertion point or an in-body reference point, or reference may be made to a position of the elongated medical device with respect to a coordinate system of the body or with respect to a coordinate system of an image or image information of the body.

Independent of the particular technique used, the detection of the position on the fiber that fulfils the particular shape information (e.g., curvature) can be used to identify the position of the elongated medical device with respect to the reference point, and as a consequence provides information regarding the length of the portion of the elongated medical device that has been inserted in the body with a resolution better than the interdistance between the gratings along the fiber. The particular shape (curvature) of the reference point thus induces a fingerprint at the position of the multicore fiber that at that moment is located at the reference point. The position of this fingerprint along the length of the multicore fiber can be detected using shape information obtained from the different gratings along the length of the optical multicore fiber, e.g., curvature information measured at the different sensor positions.

Very accurate fiber position or insertion length with respect to the reference point can be obtained by fitting or interpolating the measured shape information at the position of the reference point, e.g., fitting the curvature information at the reference point to the particular shape. By fitting or interpolating the measured shape information, the fiber position or insertion length can be determined with a higher resolution than the interdistance between the gratings, (i.e., high accuracy).

Shape and/or position of the elongated medical device is obtained from shape information obtained from gratings in the tip of the fiber as function of the total length of the multicore fiber portion that has been inserted in the body.

The latter is illustrated in FIG. 4 to FIG. 6.

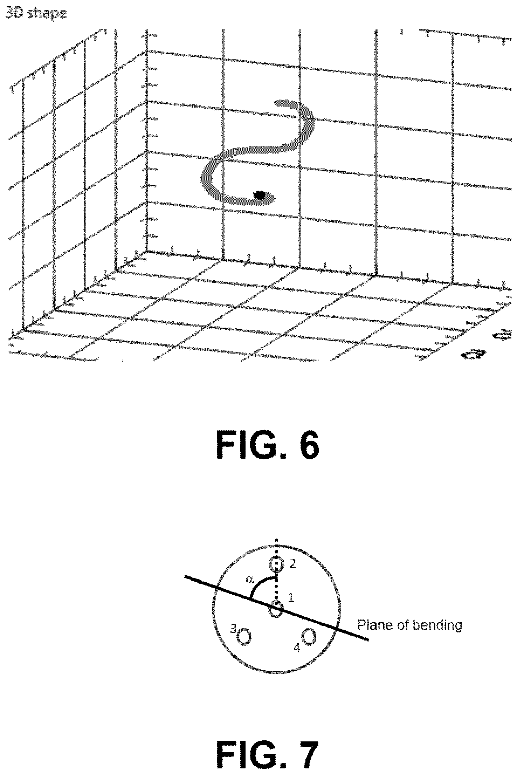

FIG. 7 illustrates a schematic overview of a cross section of the fiber that is used for the experiment. The multicore fiber used is a 4 core fiber, whereby a single core is positioned centrally and wherein 3 cores are distributed evenly around the central core. For the results shown, all three outer cores, cores 2, 3 and 4 were monitored. The fiber has a 125$\mu$m cladding and a 200$\mu$m Ormocer-T coating, comprising 38 gratings with length of 4mm and with a spacing of 1,5cm, resulting in a total sensing length of 555mm. The fiber is cut after sensor 38.

FIG. 4 illustrates a simulation of a catheter inside a blood vessel after passing a known shape. At the left of the figure, a guiding means consisting of a Teflon tube bended over a metal cylinder with a radius of 1 ,6cm, which corresponds to a curvature of 0,625 (1/cm). The guiding means goes over into a sine shaped Teflon tube which represents a blood vessel. The catheter sleeve containing the optical fiber has a total length of about 650mm with a sensing length of about 555mm and is inserted from the left side passing first the guiding means after which it enters the vessel. The catheter sleeve and multicore fiber are inserted over one period of the sine wave.

Fig. 5 shows the related curvature profile measured along the multicore fiber corresponding with the fiber inserted in the phantom vessel as shown in Fig. 4. The curvature profile in the first 160mm illustrate here the curvature occurring at the known shape near the entrance of the blood vessel and is used as a reference point for determining length information regarding the portion that has been introduced in the blood vessel. As can be seen, a curvature of about 0,625 (1/cm) can be seen at a fiber length position of 120mm, which corresponds to the position where the guiding means applies the biggest curvature to the multicore fiber. Based on this position, the inserted portion of the fiber in the body can be calculated as the total fiber length is well known. As a consequence the shape information from the tip of the fiber can be allocated to a certain position in the body.

By recording this shape information for different inserted fiber lengths in the body, the 3D shape of the path of the catheter sleeve can be reconstructed like is shown in Fig. 6. Fig. 6 shows the calculated 3D shape for the fiber corresponding to the fiber like it was inserted in Fig. 4. The small bending at the beginning of the curve shows the position of the bending means. Furthermore, the shape shows also one period of the sine wave over which the catheter sleeve containing the multicore fiber is inserted in the vessel.

By way of illustration, the principle of how to derive a bending radius, or a curvature, and bending orientation is discussed for a particular example below. Whereas the situation is discussed using a particular mathematical formalism, it is to be understood that embodiments are not limited thereto and that this is only provided for illustrating the principle. A possible principle of determining the final 3D shape or position information of a multicore fiber based on these curvatures and bending orientations is also disclosed for example in US7,813,599, and thus considered to be known as such for the person skilled in the art. The 3D shape information can for example also be calculated based on the shape information recorded from the tip of the multicore fiber as function of the total length of the multicore fiber portion that is inserted in the body.

The bending radius and direction can be derived from a shift in wavelengths of different outer gratings located at a sensor position along the fiber. The FBG wavelength shift $\Delta\lambda$ in the different cores has several contributions: (1) bending strain, (2) fiber twist, (3) longitudinal strain and (4) temperature changes. The bending strain can be calculated from the bending radius R and the distance of the core with the central plane of bending: strain = distance from plane / bending radius. The distance between the core and the bending plane can be expressed in terms of the distance d between the central core and the eccentric core together with the angle that the core makes with the central plane of bending. The distance d equals 38 $\mu$m for the used fiber in the current example but of course will depend on the fiber used, the example not being limiting for embodiments of the present invention. The angle with the central bending plane can be expressed in terms of the angle $\alpha$, which is defined as the angle between core 2 and the central plane of bending. A schematic view is shown in FIG. 7.

Let $\Delta\varepsilon_{twist}$ be the strain originating from the fiber twist, $\Delta\varepsilon_{long}$ the longitudinal strain component and $\Delta T$ the change in temperature, then the total wavelength shift $\Delta\lambda$ in each core can be expressed as follows:

$$\begin{cases} \Delta\lambda_2 = \left(\dfrac{d.\sin\alpha}{R} + \Delta\varepsilon_{twist} + \Delta\varepsilon_{long}\right).s_\varepsilon + \Delta\text{T}.s_T \\[2mm] \Delta\lambda_1 = \left(\hspace{3.2cm} \Delta\varepsilon_{long}\right).s_\varepsilon + \Delta\text{T}.s_T \\[2mm] \Delta\lambda_3 = \left(\dfrac{d.\sin(\alpha + 120°)}{R} + \Delta\varepsilon_{twist} + \Delta\varepsilon_{long}\right).s_\varepsilon + \Delta\text{T}.s_T \\[2mm] \Delta\lambda_4 = \left(\dfrac{d.\sin(\alpha + 240°)}{R} + \Delta\varepsilon_{twist} + \Delta\varepsilon_{long}\right).s_\varepsilon + \Delta\text{T}.s_T \end{cases}$$

with $s_\varepsilon$ and $s_T$ the strain respectively temperature sensitivity. This gives us 4 equations and 5 unknowns. It is clear that we can solve it for 4 independent parameters: R, $\alpha$, $\Delta\varepsilon_{twist}$ and ($\Delta\varepsilon_{long}$ $s_\varepsilon$ + $\Delta$T.$s_T$). The solution can be found based on the following goniometric relations:

$$\begin{cases} \sin(\alpha + 120°) = \sin(\alpha).\cos(120°) + \cos(\alpha).\sin(120°) \\[2mm] \qquad\qquad = \sin(\alpha).\cos(180° - 60°) + \cos(\alpha).\sin(180° - 60°) \\[2mm] \qquad\qquad = -\sin(\alpha).\cos(60°) + \cos(\alpha)\sin(60°) \\[2mm] \qquad\qquad = -\dfrac{1}{2}\sin(\alpha) + \dfrac{\sqrt{3}}{2}\cos(\alpha) \end{cases}$$

$$\begin{cases} \sin(\alpha + 240°) = \sin(\alpha - 120°) \\[2mm] \qquad\qquad = \sin(\alpha).\cos(120°) - \cos(\alpha).\sin(120°) \\[2mm] \qquad\qquad = \sin(\alpha).\cos(180° - 60°) - \cos(\alpha).\sin(180° - 60°) \\[2mm] \qquad\qquad = -\sin(\alpha).\cos(60°) - \cos(\alpha)\sin(60°) \\[2mm] \qquad\qquad = -\dfrac{1}{2}\sin(\alpha) - \dfrac{\sqrt{3}}{2}\cos(\alpha) \end{cases}$$

With these expressions, a solution can be found:

$$\begin{cases} tg\ \alpha = -\dfrac{\sqrt{3}}{3}.\left(\dfrac{2\Delta\lambda_2 - \Delta\lambda_3 - \Delta\lambda_4}{\Delta\lambda_4 - \Delta\lambda_3}\right) \\[3mm] R\ = \dfrac{\sqrt{3}d.\,s_\varepsilon}{(\Delta\lambda_3 - \Delta\lambda_4)}\cos\alpha \\[3mm] \Delta\varepsilon_{twist} = \left[\dfrac{1}{3}(\Delta\lambda_2 + \Delta\lambda_3 + \Delta\lambda_4) - \Delta\lambda_1\right]/s_\varepsilon \\[3mm] \Delta\varepsilon_{long}.s_\varepsilon + \Delta\text{T}.s_T = \Delta\lambda_1 \end{cases}$$

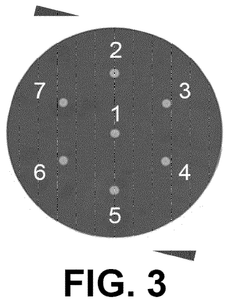

In case we have a 7-core fiber, it can sometimes be useful to use a combination of 3 other cores, not necessarily under angles of 120°. Consider for example the case of 3 adjacent outer cores, each at 60° such as for example core 2, 3 and 4 in Fig. 3.

In principle, this configuration contains the same information compared to the 120° configuration since the bending strain of the core 3 is similar in size (but opposite in sign) than the opposite core, core 6. In this case, we end up with the following set of equations:

$$\begin{cases} \Delta\lambda_2 = \left(\dfrac{d.\sin\alpha}{R} + \Delta\varepsilon_{twist} + \Delta\varepsilon_{long}\right).s_\varepsilon + \Delta\text{T}.s_T \\[2mm] \Delta\lambda_3 = \left(\dfrac{d.\sin(\alpha + 60°)}{R} + \Delta\varepsilon_{twist} + \Delta\varepsilon_{long}\right).s_\varepsilon + \Delta\text{T}.s_T \\[2mm] \Delta\lambda_4 = \left(\dfrac{d.\sin(\alpha + 120°)}{R} + \Delta\varepsilon_{twist} + \Delta\varepsilon_{long}\right).s_\varepsilon + \Delta\text{T}.s_T \end{cases}$$

For finding the solution, we can make use of the following goniometric equation:

$$\sin(\alpha + 60°) = \frac{1}{2}\sin(\alpha) + \frac{\sqrt{3}}{2}\cos(\alpha)$$

A solution can be found in a similar way like for the more symmetric case. After some math, the following solution can be found:

$$\begin{cases} tg\ \alpha = \sqrt{3}.\left(\dfrac{\Delta\lambda_3 - \Delta\lambda_4}{3\Delta\lambda_3 - \Delta\lambda_4 - 2\Delta\lambda_2}\right) \\ R = \dfrac{d.s_\varepsilon}{(\Delta\lambda_3 - \Delta\lambda_4)}\ sin\ \alpha \end{cases}$$

The above provides an illustration on how the bending radius and bending direction can be derived, and it will be understood that it is only given by way of illustration.

The present invention may make use of a comparted catheter. The comparted catheter advantageously has a central compartment, so that the optical multicore fiber can be inserted in such central compartment, such that it can be optimally used for determining shape information regarding the catheter. One example of such a catheter may be a catheter with a central compartment and with additionally one or more compartments positioned near the side of the catheter. The present invention also relates to the use of comparted catheter for obtaining shape information, as can be used in other aspects of the present invention.

In one aspect, the present invention relates to a method for determining the position of one or more points of an elongated medical device in a channel inside an object. Such an elongated medical device may for example be a catheter for blood vessels, a catheter for intubation, an endoscope, a gastroscope, a guidewire, etc. The system advantageously may be used for obtaining information regarding propagation of the elongated medical device in vessels, tracts or intestines or for obtaining information, e.g., visualising, vessels, tracts or intestines. According to some embodiments of the present invention, the method may be obtained using a system comprising a multicore fiber for insertion into the elongated medical device such that a position of the tip of the multicore fiber corresponds with a position of the tip near the tubular medical device. The multicore fiber comprises a plurality of cores. These cores may be organised as a central core surrounded by a plurality of other cores, although embodiments are not limited thereto. In some embodiments, the multicore fiber may have a central core and 2 or more cores arranged around it, e.g., 3 cores or 6 cores. The cores arranged around the central core, are also called outer cores. In embodiments of the present invention, the number of cores used in the measurement system may be different, i.e. lower, than the number of cores that is actually present in the multicore fiber. Depending on the measurements performed, different cores may be selected for use. For measuring curvature, advantageously cores are used which do not all are positioned in the same plane. According to embodiments of the present invention, in the multicore fiber, a plurality of Bragg gratings are inscribed. The plurality of Bragg gratings typically are spaced apart from each other and are positioned along the length of the multicore fiber.

The method comprises obtaining three-dimensional image data of an inside of the object comprising at least data of a channel of interest inside the object. Obtaining three-dimensional image data may be performed as described in other embodiments of the present invention. The method also comprises deriving based on the obtained three-dimensional image data derived shape information for at least part of the channel of interest. The method further comprises deriving, for positions along the channel of interest, a confinement parameter expressing whether the elongated medical device will be confined at those positions in the channel of interest. Confinement may be defined as described above.

The method also comprises obtaining measured shape information of an elongated medical device based on optical signals obtained from a multicore fiber coupled to the elongated medical device, for determining shape information of the elongated medical device.

The method finally also comprises determining a position of one or more points of the elongated medical device with respect to the three-dimensional image data or a portion thereof by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

Further features and characteristics may be as described in the summary and in other embodiments of the present invention.

In yet another aspect, the present invention also relates to a system for determining a position of an elongated medical device in an object. The system comprises a data input port for obtaining three-dimensional image data of an inside of the object comprising at least data of a channel of interest, and for obtaining optical signals obtained from a multicore fiber coupled to the elongated medical device in the object. The system also comprises a processor programmed to obtain three-dimensional image data of at least the channel of interest and for deriving based on the obtained three-dimensional image data a derived shape information for at least part of the channel of interest, and for deriving a confinement parameter expressing whether the channel of interest is confined for the elongated medical device at positions along the channel of interest. The processor furthermore is being programmed for obtaining a measured shape information of an elongated medical device based on the obtained optical signal and for determining a position of one or more points of the elongated medical device with respect to the three-dimensional image data or part thereof by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

Further features and characteristics may be as described in the summary or in other embodiments of the present invention.

In another aspect, the present invention also relates to a non-transitory computer program product adapted for, when run on a processor, performing the steps of a method for assisting in guiding an elongated medical device in an object. Such a method may comprise all steps as described in the corresponding method aspect.

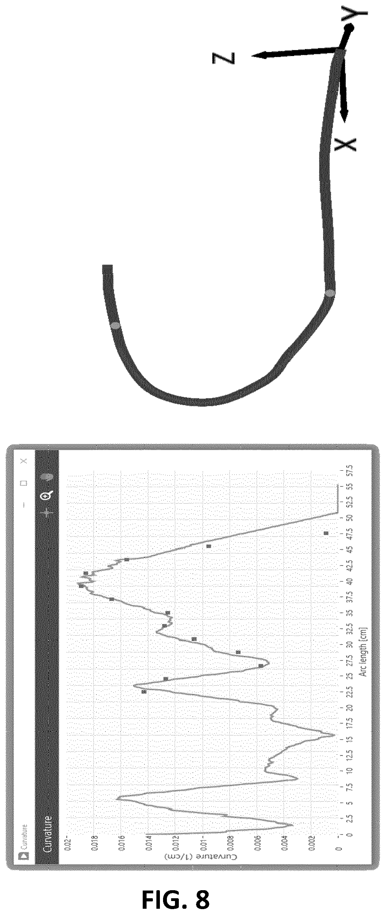

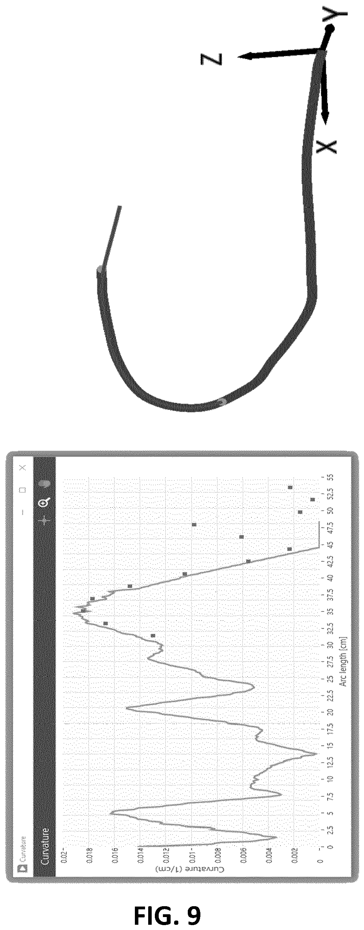

By way of illustration, embodiments of the present invention not being limited thereby, an example of an elongated medical device that is confined in a contiguous region and an example of an elongated medical device that is confined in one region but not confined in a portion corresponding with free space is shown in Fig. 8 and Fig. 9.

In one aspect, a method for determining confinement information of an elongated device for at least one position of interest in the body is disclosed. The method comprises for a plurality of conditions of the elongated device, obtaining measured shape information of the elongated device for at least one position of interest in the body based on optical signals obtained from a multicore fiber coupled to the elongated medical device. The method also comprises determining, based on a comparison of the obtained measured shape information of the elongated device at said at least one point of interest measured for the plurality of conditions, confinement information of said elongated device for that at least one position of interest in the body. In some embodiments, a variation in the obtained measured shape information may be quantified and based thereon the confinement information is determined Quantification may comprise determining a variance, a standard deviation, a difference between extremes of a value expressing shape information. The value expressing shape information may be curvature, bending, Measurements under a plurality of conditions may refer to measurements recorded at different moments in time, optionally after movement of the elongated device, and/or measurements performed during insertion or withdrawal of the elongated device, using e.g. optical signals stemming from different positions along the elongated device corresponding with said at least one position of interest in the body.

Where in embodiments of the present invention reference is made to shape information, this may in some embodiments refer to curvature information, such as for example bending direction, curvature direction, curvature angle, or another parameter describing a curvature profile or a curvature profile. It may in some examples be curvature information such as a bending direction or curvature direction along the arc length of the path or along the curved path. In some embodiments, shape information may comprise or may be a 3D shape, such as for example 3D coordinates.

The method furthermore may comprise, based on said determined confinement information, indicating a degree of confinement of different points along the elongated device with respect to the body. Further features and advantages may as set out in the summary or other aspects of the present invention.

The present invention also relates to a system for determining confinement information of an elongated device for at least one position of interest in the body, with a corresponding data input port and a corresponding processor, performing or being adapted for performing steps of the corresponding method. Furthermore a graphical user interface for outputting confinement information of the elongated device for one or more positions in the body also is described. Also a corresponding non-transitory computer program product adapted for, when run on a processor, performing the steps of the method for determining confinement information as described above is envisaged.

## Claims

1. A method for determining the position of one or more points of an elongated medical device in a channel inside an object, the method comprising

  - obtaining three-dimensional image data of an inside of the object comprising at least data of a channel of interest inside the object,
  - deriving based on the obtained three-dimensional image data derived shape information for at least part of the channel of interest,
  - deriving, for positions along the channel of interest, a confinement parameter expressing whether the elongated medical device will be confined at those positions in the channel of interest,
  - obtaining measured shape information of an elongated medical device based on optical signals obtained from a multicore fiber coupled to the elongated medical device, for determining shape information of the elongated medical device, and
- determining a position of one or more points of the elongated medical device with respect to the three-dimensional image data or a portion thereof by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

2. A method according to claim 1, the method furthermore comprising, based on said determining a position, showing said determined position of one or more points of the elongated medical device with respect to the three-dimensional image data.

3. A method according to any of the previous claims, wherein deriving a confinement parameter comprises indicating that the channel of interest is confined for positions where a channel width is smaller than four times the width of the elongated medical device and selecting these portions for use.

4. A method according to claim 3, wherein deriving a confinement parameter based on the channel width is performed using the three-dimensional image data.

5. A method according to any of the previous claims, wherein at positions along the channel of interest where the channel of interest is not confined according to the confinement parameter, the orientation of the elongated medical device is derived from the measured shape information measured using the multicore fiber.

6. A method according to any of the previous claims, wherein said determining a position of one or more points of the elongated medical device comprises determining a position of one or more points of the elongated medical device based on said mapping for the portion of the channel of interest wherein the elongated medical device is confined and determining a position of one or more additional points of the elongated medical device for points where the elongated medical device is not confined in the channel of interest from the measured shape information.

7. A method according to claim 6, wherein the portion of the channel of interest wherein the elongated medical device is confined is a single contiguous portion, and whereby a portion of the channel of interest wherein the elongated medical device is not confined, corresponds to free space.

8. A method according to claim 7, wherein the method comprises determining a position of the end point of the elongated medical device being in free space, said determining being based on mapping of a portion of the elongated medical device where it is confined in the channel of interest and on the measured shape information for the portion of the elongated medical device where it is not confined.

9. A method according to any of the previous claims, wherein the portion of the channel of interest wherein the elongated medical device is confined corresponds with two or more non-contiguous regions.

10. A method according to any of the previous claims, wherein obtaining the derived shape information of a portion of a channel of interest and/or obtaining the measured shape information of a portion of an elongated medical device comprises obtaining shape information as function of a length along that portion of a channel of interest or along that portion of an elongated medical device.

11. A method according to any of the previous claims, wherein determining a position of one or more points of the elongated medical device with respect to the three-dimensional image data comprises

- determining, for a plurality of channels of the three-dimensional image data, the difference between at least a portion of the measured shape information and at least a portion of the derived shape information of the channel,
- identifying the channel of the three-dimensional image data showing the smallest difference between the at least portion of the measured shape information and the at least portion of the derived shape information, and
- matching the position of the elongated medical device with the position of the identified channel.

12. A method according to claim 11, wherein the plurality of channels represent different branches of a branched channel of interest.

**13.** A system for determining a position of an elongated medical device in an object, the system comprising

- a data input port for obtaining three-dimensional image data of an inside of the object comprising at least data of a channel of interest, and for obtaining optical signals obtained from a multicore fiber coupled to the elongated medical device in the object,
- a processor programmed to obtain three-dimensional image data of at least the channel of interest and for deriving based on the obtained three-dimensional image data a derived shape information for at least part of the channel of interest, and for deriving a confinement parameter expressing whether the channel of interest is confined for the elongated medical device at positions along the channel of interest,
- the processor being programmed for obtaining a measured shape information of an elongated medical device based on the obtained optical signals,
- the processor furthermore being programmed for determining a position of one or more points of the elongated medical device with respect to the three-dimensional image data or part thereof by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

**14.** The system according to claim 13, the system furthermore comprising a graphical user interface for outputting a position of one or more points of the elongated medical device with respect to at least part of the three-dimensional image.

**15.** A non-transitory computer program product adapted for, when run on a processor, performing the steps of a method for assisting in guiding an elongated medical device in an object, the method comprising

- obtaining three-dimensional image data of an inside of the object comprising at least data of a channel of interest,
- deriving based on the obtained three-dimensional image data a derived shape information for at least part of the channel of interest,
- obtaining measured shape information of an elongated medical device based on optical signals obtained from a multicore fiber coupled to the elongated medical device in the object, for the elongated medical device when inserted in the object, and
- determining a position of one or more points of the elongated medical device with respect to the three-dimensional image data by mapping a portion of the measured shape information of the elongated medical device with a portion of the derived shape information for at least part of the channel of interest, the portion of the derived shape information corresponding with positions where the elongated medical device is confined in the channel of interest, based on the confinement parameter.

Fiber in the tubular shaped
medical device with induced
predetermined shape

Guiding means

# FIG. 1

inserter

Induced curvature

body

Fiber in
catheter

Blood
vessel

# FIG. 2

FIG. 3

# FIG. 4

# FIG. 5

3D shape

# FIG. 6

# FIG. 7

**FIG. 8**

FIG. 9

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/303890 A1 (DUINDAM VINCENT [US] ET AL) 14 November 2013 (2013-11-14) * paragraphs [0026], [0027], [0034] - [0057] * ----- | 1-15 | INV. A61B5/06 A61B34/20 |
| A | WO 2012/158324 A2 (INTUITIVE SURGICAL OPERATIONS [US]) 22 November 2012 (2012-11-22) * paragraphs [0032] - [0051] * ----- | 1-15 | |
| A | US 2022/079683 A1 (BYDLON TORRE MICHELLE [US] ET AL) 17 March 2022 (2022-03-17) * paragraphs [0061] - [0069] * ----- | 1-15 | |
| | | | TECHNICAL FIELDS SEARCHED (IPC)  A61B |

The present search report has been drawn up for all claims

| Place of search | Date of completion of the search | Examiner |
|---|---|---|
| Berlin | 16 October 2024 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 7641

This annex lists the patent family members relating to the patent documents cited in the above-mentioned European search report.
The members are as contained in the European Patent Office EDP file on
The European Patent Office is in no way liable for these particulars which are merely given for the purpose of information.

16-10-2024

| Patent document cited in search report | Publication date | Patent family member(s) | | Publication date |
|---|---|---|---|---|
| US 2013303890 A1 | 14-11-2013 | CN | 104302241 A | 21-01-2015 |
| | | CN | 109452930 A | 12-03-2019 |
| | | EP | 2849670 A1 | 25-03-2015 |
| | | EP | 3524184 A1 | 14-08-2019 |
| | | JP | 6386998 B2 | 05-09-2018 |
| | | JP | 2015519130 A | 09-07-2015 |
| | | KR | 20150017326 A | 16-02-2015 |
| | | US | 2013303890 A1 | 14-11-2013 |
| | | US | 2013303894 A1 | 14-11-2013 |
| | | US | 2019175060 A1 | 13-06-2019 |
| | | US | 2019320937 A1 | 24-10-2019 |
| | | US | 2022151508 A1 | 19-05-2022 |
| | | US | 2024049980 A1 | 15-02-2024 |
| | | WO | 2013173227 A1 | 21-11-2013 |
| WO 2012158324 A2 | 22-11-2012 | CN | 103648361 A | 19-03-2014 |
| | | CN | 105919547 A | 07-09-2016 |
| | | EP | 2709512 A2 | 26-03-2014 |
| | | EP | 3058889 A1 | 24-08-2016 |
| | | KR | 20140033128 A | 17-03-2014 |
| | | US | 2012289777 A1 | 15-11-2012 |
| | | US | 2015148690 A1 | 28-05-2015 |
| | | WO | 2012158324 A2 | 22-11-2012 |
| US 2022079683 A1 | 17-03-2022 | US | 2022079683 A1 | 17-03-2022 |
| | | WO | 2020002176 A2 | 02-01-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3934504 A
- EP 4233764 A
- US 20140053654 A
- US 7813599 B